# EUROPÄISCHE PATENTANMELDUNG

(11) **EP 0 573 829 A1**
(43) Veröffentlichungstag der Anmeldung: **15.12.1993**
(21) Anmeldenummer: 93108240.8
(22) Anmeldetag: 21.05.1993
(51) Int. Cl.: C07D 498/04, A61K 31/505

(54) **Pyrimidofuroxane und ihre Verwendung als pharmakologische Wirkstoffe**

(30) Priorität: 10.06.1992 DE 4218979
(71) Anmelder: HOECHST AKTIENGESELLSCHAFT, D-65926 Frankfurt am Main (DE)
(72) Erfinder: Brendel, Joachim, Dr., D-61118 Bad Vilbel (DE); Schönafinger, Karl, Dr., W-8755 Alzenau (DE); Bohn, Helmut, Dr., W-6369 Schöneck 1 (DE)

(57) **Zusammenfassung**

Die vorliegende Erfindung betrifft Pyrimidofuroxane der allgemeinen Formel I 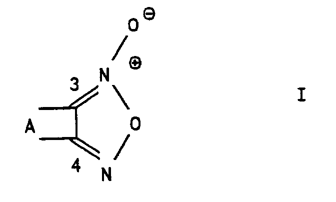
worin 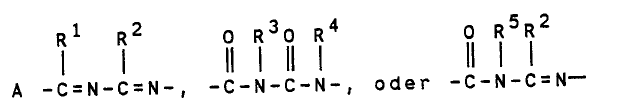
bedeutet, wobei jeweils das Stickstoffatom über das C-4 und das Kohlenstoffatom über das C-3 des Furoxanrings gebunden ist und die Reste R wie in Anspruch 1 angegeben definiert sind, Verfahren zu ihrer Herstellung und ihre Verwendung.

## Beschreibung

Die vorliegende Erfindung betrifft Pyrimidofuroxane, ihre Herstellung und ihre Verwendung als pharmakologische Wirkstoffe.

Eine Reihe von Verbindungen aus der Klasse der Pyrimidofuroxane ist bereits bekannt und insbesondere in der wisschenschaftlichen Literatur beschrieben (siehe z.B. Arzneim.-Forsch. 33 (1983) 803; J. Chem. Soc., Perkin Trans I 1976, 1327; J. Org. Chem. 1968, 2086; J. Chem. Soc., Chem. Comm. 1982, 267 und 60; Heterocycles 1975, 113; J. Het. Chem. 1973, 993 sowie DE-OS 27 03 369). Als pharmakologische Wirkstoffe sind bisher lediglich unkondensierte, substituierte Furoxane beschrieben (siehe z.B.: EP-B 0 038 438, EP-B 0 054 872 und EP-B 0 054 873).

Die vorliegende Erfindung betrifft Pyrimidofuroxane der allgemeinen Formel I 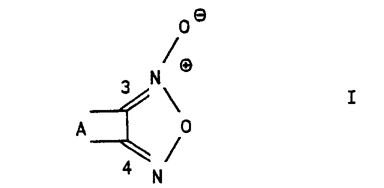
worin 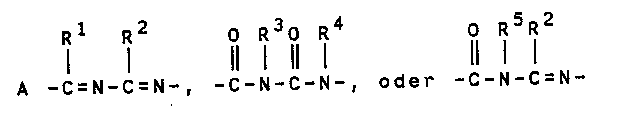
bedeutet, wobei jeweils das Stickstoffatom über das C-4 und das Kohlenstoffatom über das C-3 des Furoxanrings gebunden ist;
R¹ -NHR⁶, -NR⁷R⁸, -N X, -NHOH, -NH(CH₂)ₙY,
-N[(CH₂)ₙY]₂ oder -NH(CH₂)ₘZ bedeutet;
R² Wasserstoff oder -OR⁷ bedeutet;
R³ (C₂-C₄)-Alkyl bedeutet;
R⁴ Wasserstoff, (C₁-C₆)-Alkyl, (C₅-C₇)-Cycloalkyl,
-CH₂Z, -CH₂COOR⁷, -CH₂CONR⁷R⁸, -CH₂CON X oder
-(CH₂)ₙY bedeutet;
R⁵ eine der Bedeutungen von R⁴ mit Ausnahme von Wasserstoff hat;
R⁶ (C₁-C₆)Alkyl oder (C₅-C₇)-Cycloalkyl bedeutet;
R⁷ und R⁸ unabhängig voneinander (C₁-C₄)Alkyl bedeutet;
X -(CH₂)ₚ-, -(CH₂)₂-O-(CH₂)₂- oder 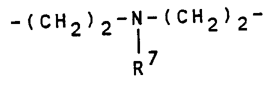
bedeutet;
Y -OH, -OR⁷ oder -NR⁷R⁸ bedeutet;
Z Aryl oder Heteroaryl, die gegebenenfalls auch substituiert sein können, bedeutet;
n für 2, 3 oder 4 steht;
m für 1 oder 2 steht; und
p für 4, 5 oder 6 steht,
sowie deren pharmakologisch annehmbare Säureadditionsverbindungen.

Die vorliegende Erfindung betrifft demnach Verbindungen der allgemeinen Formel Ia 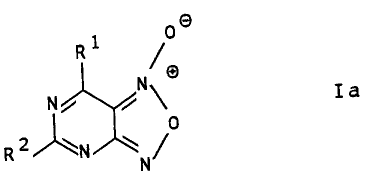
der allgemeinen Formel Ib 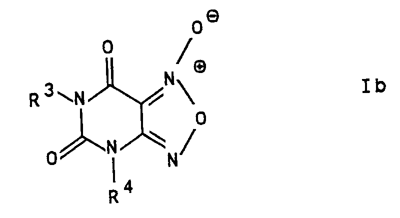
sowie der allgemeinen Formel Ic 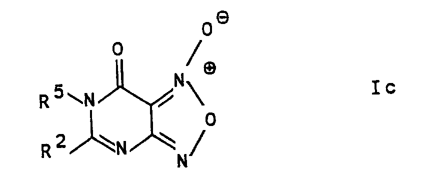
wobei jeweils die Reste R¹ bis R⁵ wie oben angegeben definiert sind.

Für R³, R⁴, R⁵, R⁶ sowie R⁷ und R⁸ stehende (C₂-C₄)-, (C₁-C₆)- bzw. (C₁-C₄)-Alkylreste können geradkettig oder verzweigt sein. Beispiele für solche Alkylreste sind Methyl, Ethyl, n-Propyl, i-Propyl, n-Butyl, i-Butyl, sek-Butyl, tert.-Butyl sowie Pentyl und Hexyl.

Für R⁴, R⁵ oder R⁶ stehendes (C₅-C₇)-Cycloalkyl ist insbesondere Cyclopentyl, Cyclohexyl und Cycloheptyl.

Für Z stehendes Aryl ist insbesondere (C₆-C₁₄)-Aryl, wobei Phenyl bevorzugt ist.

Für Z stehendes Heteroaryl ist bevorzugt 5- bis 7-gliedrig und leitet sich beispielsweise von Pyrrol oder Pyridin ab. Bevorzugt sind α-Pyridyl und β-Pyridyl.

Für Z stehendes Aryl und Heteroaryl können auch substituiert sein. Geeignete Substituenten sind beispielsweise (C₁-C₄)-Alkyl, (C₁-C₄)-Alkoxy, Amino, (C₁-C₄)-Alkylamino, Di-(C₁-C₄)-alkylamino, (C₁-C₆)-Alkanoylamino, Halogen, vorzugsweise Fluor, Chlor oder Brom, Hydroxy, Nitro oder Cyano. Aryl oder Heteroaryl können durch die genannten Substituenten auch mehrfach, beispielsweise zwei- oder dreifach substituiert sein.

Ein bevorzugter substituierter Arylrest ist 3,4-Dimethoxyphenyl.

Von den Resten R¹, R⁴ und R⁵ sind diejenigen bevorzugt, die basische oder hydrophile Gruppen enthalten. Für R⁴ ist zusätzlich auch Wasserstoff bevorzugt.

Besonders bevorzugte Reste R¹ sind 2-(Pyridylmethyl)amino, 3-(Pyridylmethyl)amino, 2-Hydroxyethylamino, 2-Methoxyethylamino, 2-Diethylaminoethylamino, 2-(3,4-Dimethoxyphenyl)ethylamino, n-Butylamino, N,N-Di-(hydroxyethyl)amino, N-Methylpiperazino und Morpholino.

Besonders bevorzugte Reste R⁴ sind Wasserstoff, Methyl, Benzyl, Ethoxycarbonylmethyl, Dimethylaminocarbonylmethyl, Morpholinocarbonylmethyl, 2-Pyridylmethyl und 3-Pyridylmethyl.

Besonders bevorzugte Reste R⁵ sind die besonders bevorzugten Reste R⁴ mit Ausnahme von Wasserstoff.
R² bedeutet bevorzugt Wasserstoff, Methoxy oder Ethoxy.
R³ bedeutet bevorzugt Ethyl.

Ganz besonders bevorzugte erfindungsgemäße Verbindungen der allgemeinen Formel I sind 6-Ethyl-[1,2,5]oxadiazolo[3,4-d]pyrimidin-5,7(4H,6H)-dion-1-oxid sowie 5-Methoxy-7-[(3-pyridylmethyl)-amino]-[1,2,5]oxadiazolo [3,4-d]pyrimidin-1-oxid.

Erfindungsgemäße Verbindungen der allgemeinen Formel Ia können beispielsweise dadurch hergestellt werden, daß Verbindungen der allgemeinen Formel II 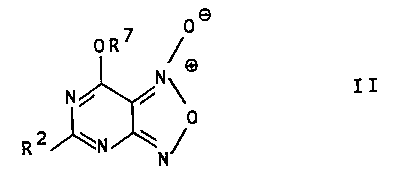
worin R² und R⁷ wie oben angegeben definiert sind, mit Aminen der allgemeinen Formel HR¹, worin R¹ wie oben angegeben definiert ist, umgesetzt werden. Dabei wird die Alkoxygruppe -OR⁷ durch die Aminogruppe -R¹ substituiert.

Die Reaktion wird zweckmäßigerweise in einem organischen Lösungs- oder Dispergiermittel wie niederen Alkoholen, Ethern oder Estern, bevorzugt in Methanol oder Ethanol, durchgeführt. Die Umsetzung läuft nach Zugabe der stöchiometrischen Menge des Amins bei Temperaturen von 0 bis 60^{o}C ab. Vorzugsweise wird die Umsetzung bei 20 bis 40^{o}C durchgeführt.

Die Verbindungen der allgemeinen Formel II sind entweder bekannt (beispielsweise für R²=H und R⁷=Methyl; Binder et al., Arzneim.-Forsch. 33 (1983) 803; für R²=Methoxy und R⁷=Methyl; Nutiu, Boulton, J. Chem. Soc. Perkin Trans. 1, 1976, 1327) oder können analog bekannten Verfahren hergestellt werden. Beispielsweise kann gemäß folgendem Schema aus 2,4,6-Trichlorpyrimidin der Formel III durch Reaktion mit zwei Äquivalenten eines Alkalimetallalkoholates die Verbindung der allgemeinen Formel IV erhalten werden, die wiederum zur Verbindung der allgemeinen Formel V nitriert werden kann. Umsetzung mit Hydrazin führt zur Verbindung der allgemeinen Formel VI. Diese kann schließlich durch Umsetzung mit Natriumnitrit in wäßriger Salzsäure gefolgt von Erhitzen in einem Lösungsmittel wie Tetrahydrofuran, Chloroform oder Toluol auf 65 bis 110^{o}C unter Stickstoffabspaltung in Verbindungen der allgemeinen Formel IIa überführt werden. Alternativ können die Verbindungen der allgemeinen Formel V auch durch Erhitzen mit Natriumazid in Tetrahydrofuran direkt zu Verbindungen der allgemeinen Formel IIa umgesetzt werden. 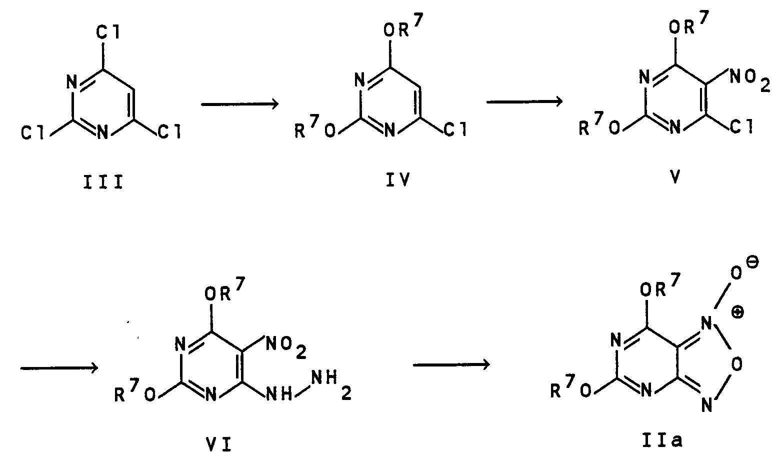
Verbindungen der allgemeinen Formel Ib können beispielsweise gemäß folgendem Schema aus Verbindungen der allgemeinen Formel VII durch Erhitzen in einem hochsiedenden organischen Lösungsmittel, wie beispielsweise Toluol, Xylol, Dioxan oder Dimethylformamid, vorteilhafterweise in Gegenwart von 10 % Essigsäure, bei Temperaturen von 90 bis 150^{o}C erhalten werden. Die dabei zunächst entstehenden Verbindungen der allgemeinen Formel Ib/1 können gegebenenfalls in erfindungsgemäße Verbindungen der allgemeinen Formel Ib/2, worin R^{4'} mit Ausnahme von Wasserstoff wie R⁴ definiert ist, überführt werden durch basenkatalysierte Alkylierung mit Verbindungen der allgemeinen Formel R^{4'}Hal, worin Hal Chlor, Brom, Iod, Mesylat oder Tosylat bedeutet.

Diese Alkylierung wird vorteilhafterweise in einem polaren organischen Lösungsmittel wie z.B. Tetrahydrofuran, Dimethoxyethan, Dimethylformamid, Dimethylsulfoxid, Methanol, Ethanol, Acetonitril oder Gemischen dieser Lösungsmittel, bevorzugt in Tetrahydrofuran oder Dimethylformamid durchgeführt. Als Base können Alkalimetallhydride, wie z.B. Natriumhydrid und Lithiumhydrid oder Alkalicarbonate, wie z.B. Kaliumcarbonat, verwendet werden, vorzugsweise in molarer Menge. Die Reaktion wird im allgemeinen bei 40 bis 150^{o}C, vorzugsweise bei 50 bis 100^{o}C, durchgeführt. 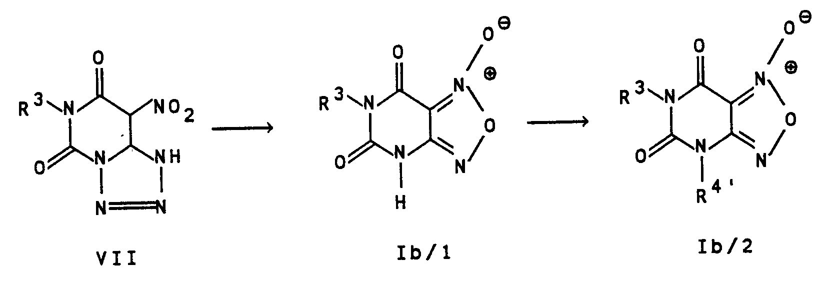
Die Verbindungen der allgemeinen Formel VII können beispielsweise auf folgendem, im Prinzip bekannten Wege hergestellt werden:

Aus 1-Alkyl-4-chloruracilen der allgemeinen Formel VIII (siehe DE-P 11 39 505) werden zunächst durch Nitrierung die Verbindungen der allgemeinen Formel IX erhalten, deren Umsetzung mit Natriumazid die Nitrotetrazolopyrimidine der allgemeinen Formel VII (bzw. die entsprechenden isomeren Nitroazidopyrimidine) liefert. 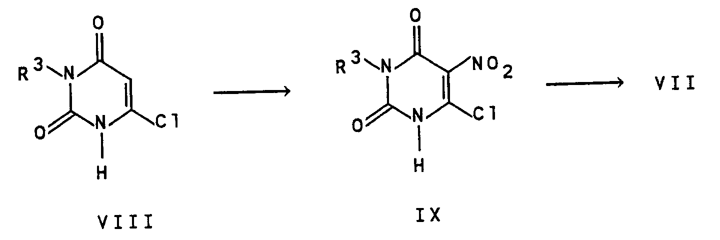
Zur Synthese von Verbindungen der allgemeinen Formel Ib/2 können aber auch die Verbindungen der allgemeinen Formel VIII zunächst zu den 1,3-Dialkyl-4-chlor-uracilen der allgemeinen Formel X 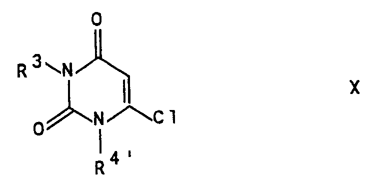
umgesetzt (Pfleiderer et al., Chem. Ber. 111 (1978) 982) und dann wie oben beschrieben nitriert, mit Natriumazid substituiert und erhitzt werden.

Verbindungen der allgemeinen Formel Ic können beispielsweise aus Verbindungen der allgemeinen Formel XI 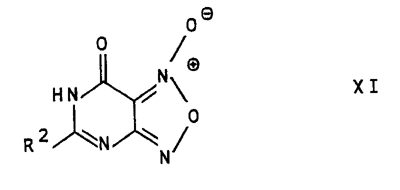
durch basenkatalysierte Alkylierung mit Verbindungen der allgemeinen Formel R⁵Hal, worin R⁵ wie oben angegeben definiert ist und Hal Chlor, Brom, Iod, Mesylat oder Tosylat bedeutet, hergestellt werden.

Diese Alkylierung wird vorteilhafterweise in einem polaren organischen Lösungsmittel wie z.B. Tetrahydrofuran, Dimethoxyethan, Dimethylformamid, Dimethylsulfoxid, Methanol, Ethanol, Acetonitril oder Gemischen dieser Lösungsmittel, bevorzugt in Tetrahydrofuran durchgeführt. Als Base können Alkalimetallhydride, wie z.B. Natriumhydrid und Lithiumhydrid oder Alkalicarbonate, wie z.B. Kaliumcarbonat, verwendet werden, bevorzugt wird Lithiumhydrid. Die Reaktion wird im allgemeinen bei 20 bis 150^{o}C, vorzugsweise bei 20 bis 65^{o}C, durchgeführt.

Die Verbindung der allgemeinen Formel XI, in der R² Wasserstoff bedeutet ist bekannt und bei Temple et al. J. Org. Chem. 33 (1968) 2086 beschrieben. Entsprechende Verbindungen, in denen R² für -OR⁷ steht, können hergestellt werden aus Verbindungen der allgemeinen Formel XII 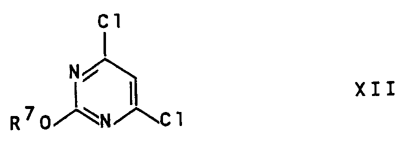
durch Substitution eines Chloratoms gegen eine Hydroxygruppe, anschließende Nitrierung und Umsetzung mit Natriumazid.

Die Verbindungen der allgemeinen Formel XII ihrerseits können durch Umsetzung der Verbindung der Formel XIII 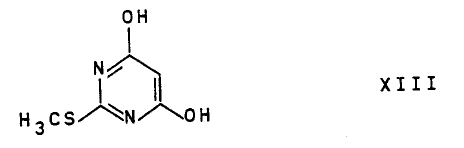
mit Natriummethylat und Phosphoroxychlorid oder aus der Verbindung der Formel III mit Natriummethylat hergestellt werden.

Erfindungsgemäße Verbindungen der allgemeinen Formel I, die in den Resten R¹, R⁴ oder R⁵ eine basische Gruppe enthalten, können mit anorganischen oder organischen Säuren Salze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure-(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Die Verbindungen der allgemeinen Formel I und ihre pharmakologisch annehmbaren Säureadditionssalze besitzen wertvolle pharmakologische Eigenschaften. Am Modell der Kalium-depolarisierten Pulmonalarterie des Meerschweinchen führen sie in niedrigen Konzentrationen zu einer Relaxation. Diese Wirkung kann mit Oxyhämoglobin inhibiert werden, was auf einen NO-mediierten Mechanismus deutet. Stickstoffmonoxid führt als Aktivator der Guanylatcyclase zu einer Erhöhung von cyclischem Guanosinmonophosphat, welches im glatten Muskel eine Relaxation und in den Blutplättchen antiadhäsive und antiaggregatiorische Wirkungen verursacht. Stickstoffmonoxid ist außerdem auch entscheidend beteiligt bei Lernvorgängen, bei der Regulation der Nierenfunktion, bei der Immunabwehr, beim septischen Schock und bei erektilen Dysfunktionen. Die Verbindungen der allgemeinen Formel I und ihre pharmakologisch annehmbaren Säureadditionssalze können somit bei den genannten Indikationen eingesetzt werden. Vor allem aber haben sich NO-Donoren zur Behandlung und Prophylaxe von angina pectoris bewährt.

Die Verbindungen der allgemeinen Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der allgemeinen Formel I, ihre pharmakologisch annehmbaren Säureadditionsssalze und pharmazeutische Präparate, welche die Verbindungen der allgemeinen Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Darüber hinaus können sie auch zur Behandlung erektiler Dysfunktionen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

### Beispiel 1:

### 7-Morpholino-[1,2,5]oxadiazolo[3,4-d]pyrimidin-1-oxid

Zu einer Mischung von 3,0 g (18 mmol) 7-Methoxy-[1,2,5]oxadiazolo[3,4-d]pyrimidin-1-oxid (Arzneim.-Forsch. 33 (1983) 803) und 100 ml Ethanol wird eine Lösung vom 1,6 g (18 mmol) Morpholin in 20 ml Ethanol zugefügt. Nach 4 Stunden Rühren bei Raumtemperatur wird das Lösungsmittel im Vakuum abdestilliert und der Rückstand aus Isopropanol umkristallisiert. Man erhält 3,6 g (90 %) 7-Morpholino-[1,2,5]oxadiazolo-[3,4-d]pyrimidin-1-oxid.
Fp. 145 - 147^{o}C

### Beispiel 2:

### 7-(2-Methoxyethylamino)-[1,2,5]oxadiazolo-[3,4-d]pyrimidin-1-oxid

Zu einer Suspension von 4,0 g (24 mmol) 7-Methoxy-[1,2,5]oxadiazolo[3,4-d]pyrimidin-1-oxid in 120 ml Methanol wird eine Lösung von 1,8 g (24 mmol) Methoxyethylamin in 5 ml Methanol zugetropft. Das Ausgangsmaterial geht in Lösung und gleich darauf fällt das Produkt aus der Reaktionsmischung aus. Nach 30 Minuten wird der Niederschlag abgesaugt und mit Methanol gewaschen. Man erhält 3,4 g (68 %) 7-(2-Methoxyethylamino)-[1,2,5]oxadiazolo [3,4-d]pyrimidin-1-oxid.
Fp. 135 - 137^{o}C (Ethanol)

### Beispiel 3:

### 7-(2-Diethylaminoethylamino)-5-methoxy-[1,2,5]-oxadiazolo[3,4-d]pyrimidin-1-oxid

Eine Mischung aus 3,0 g (15 mmol) 5,7-Dimethoxy-[1,2,5]oxadiazolo[3,4-d]pyrimidin-1-oxid (J. Chem. Soc. Perkin Trans. 1, 1976, 1327), 1,75 g (15 mmol) Diethylaminoethylamin und 120 ml Methanol wird 2 Stunden bei Raumtemperatur gerührt und dann am Rotationsverdampfer eingeengt. Der Rückstand wird heiß in Isopropanol gelöst, abfiltriert und mit der gleichen Menge heißen Petrolethers versetzt. Über Nacht kristallisieren 2,5 g (59 %) 7-(2-Diethylaminoethylamino)-5-methoxy-[1,2,5]oxadiazolo[3,4-d]pyrimidinlo-1-oxid aus.
Fp. 105 - 107^{o}C

### Beispiel 4:

### 5-Methoxy-7-(3-picolylamino)-[1,2,5]oxadiazolo-[3,4-d]pyrimidin-1-oxid

Aus 3,0 g (15 mmol) 5,7-Dimethoxy-[1,2,5]oxadiazolo[3,4-d]pyrimidin-1-oxid und 1,6 g (15 mmol) 3-Picolylamin werden analog Beispiel 3 0,75 g (18 %) 5-Methoxy-7-(3-picolylamino)-[1,2,5]oxadiazolo-[3,4-d]pyrimidin-1-oxid erhalten.
Fp. 136 - 138^{o}C

### Beispiel 5:

### 6-Ethyl-[1,2,5]oxadiazolo[3,4-d]pyrimidin-5,7(4H,6H)-dion-1-oxid

a) 67,5 g (0,39 mol) 1-Ethyl-4-chloruracil (Deutsche Patentschrift Nr. 11 39 505) werden unter Eiskühlung portionsweise in 360 g konzentrierte Schwefelsäure eingetragen. Man tropft 70 ml rauchende Salpetersäure bei 5 - 10^{o}C zu, rührt noch 2 Stunden bei 5^{o}C nach und gibt das Reaktionsgemisch dann auf 700 g Eis. Nach zweistündigem Rühren wird das ausgefallene Produkt abgesaugt, mit Wasser gewaschen und im Vakuum getrocknet. Man erhält 63 g (74 %) 1-Ethyl-4-chlor-5-nitrouracil.
   Fp. 182^{o}C
b) 61,5 g (0,28 mol)1-Ethyl-4-chlor-5-nitrouracil werden mit 36,4 g (0,56 mol) Natriumazid in 3500 ml THF 7 Stunden unter Rückfluß erhitzt. Man läßt über Nacht stehen, saugt den ausgefallenen Niederschlag (90 g) ab und rührt 1 Stunde mit 850 ml Aceton. Einengen des Acetonextraktes liefert 53 g (84 %) 6-Ethyl-8-nitrotetrazolo-[1,5-c]pyrimidin-5,7(1H,6H)-dion.
c) 20,0 g 6-Ethyl-8-nitrotetrazolo[1,5-c]pyrimidin-5,7(1H,6H)-dion werden in 2000 ml Xylol mit 200 ml Eisessig 7 Stunden auf 120^{o}C erhitzt. Ein geringer ungelöster Teil wird abfiltriert, das Filtrat wird im Vakuum eingeengt und der ölige Rückstand wird durch Verreiben mit Methylenchlorid zur Kristallisation gebracht. Man erhält 13,5 g (77 %) 6-Ethyl-[1,2,5]oxadiazolo-[3,4-d]pyrimidin-5,7(4H,6H)-dion-1-oxid.
   Fp. 141 - 142^{o}C

### Beispiel 6:

### 6-Ethyl-4-morpholinocarbonylmethyl-[1,2,5]oxadiazolo-[3,4-d]pyrimidin-5,7(4H,6H)-dion-1-oxid

a) Zu einer Lösung von 58 g (0,28 mol) Bromacetylbromid in 500 ml THF werden 50 g (0,57 mol) Morpholin bei 10^{o}C zugetropft. Nach 2 Stunden wird vom ausgefallenen Morpholinhydrobromid abfiltriert, eingeengt und im Vakuum bei 110^{o}C/0,5 Torr destilliert. Man erhält 39 g (67 %) Bromacetylmorpholid als farblose Kristalle, die wenig oberhalb Raumtemperatur schmelzen.
b) Zu einer Lösung von 5,0 g (25 mmol) 6-Ethyl-[1,2,5]oxadiazolo[3,4-d]pyrimidin-5,7(4H,6H)-dion-1-oxid in 150 ml DMF werden 0,3 g (38 mmol) Lithiumhydrid portionsweise zugefügt. Man erhitzt 30 Minuten auf 60^{o}C und tropft dann eine Lösung von 8,0 g (38 mmol) Bromacetylmorpholid in 50 ml DMF zu. Nach weiteren 90 Minuten bei 60^{o}C wird das DMF am Rotationsverdampfer weitgehend abdestilliert, der Rückstand mit Wasser versetzt und mit Essigester extrahiert. Der Essigesterextrakt wird bis zur beginnenden Kristallisation eingeengt und man erhält 3,0 g (37 %) 6-Ethyl-4-morpholino-carbonylmethyl-[1,2,5]oxadiazolo-[3,4-d]pyrimidin-5,7(4H,6H)-dion-1-oxid.
   Fp. 205^{o}C

### Beispiel 7:

### 6-Ethyl-4-(3-pyridylmethyl)-[1,2,5]oxadiazolo-[3,4-d]pyrimidin-5,7(4H,6H)-dion-1-oxid

Zu einer Lösung von 5,0 g (25 mmol) 6-Ethyl-[1,2,5]oxadiazolo[3,4-d]pyrimidin-5,7(4H,6H)-dion-1-oxid in 250 ml DMF werden 0,48 g (60 mmol) Lithiumhydrid portionsweise zugefügt. Man erhitzt 15 Minuten auf 60^{o}C, fügt dann 4,9 g (30 mmol) 3-Picolylchlorid Hydrochlorid zu und erhitzt 3 Stunden auf 80^{o}C. Dann engt man weitgehend ein, nimmt mit Essigester auf und extrahiert mit 10 %iger Salzsäure. Der wäßrige Extrakt wird neutralisiert und mit Essigester extrahiert. Leichterflüchtige Verunreinigungen werden im Vakuum bis 100^{o}C/0,5 Torr abdestilliert und der Rückstand wird chromatographiert. Man erhält 1,4 g (20 %) 6-Ethyl-4-(3-pyridylmethyl)-[1,2,5]oxadiazolo[3,4-d]pyrimidin-5,7(4H,6H)-dion-1-oxid als hochviskoses Öl.
¹³C-NMR (DMSO): δ = 12,7 (q), 37,0 (t), 44,1 (t), 101,6 (s), 123,7 (d), 130,7 (s), 135,7 (d), 149,1 (d), 149,2 (d), 149,3 (s), 151,6 (s), 151,6 (s)

### Beispiel 8:

### 6-Methyl-[1,2,5]oxadiazolo[3,4-d]pyrimidin-7(6H)-on-1-oxid

Eine Lösung von 3,0 g (20 mmol) 7-Hydroxy-[1,2,5]oxadiazolo[3,4-d]pyrimidin-1-oxid (C. Temple, C. L. Kussner, J. A. Montgomery, J. Org. Chem. 33 (1968) 2086) in 30 ml THF wird zu einer Suspension von 0,18 g (22,5 mmol) Lithiumhydrid in 20 ml THF zugetropft. Man erwärmt 30 Minuten auf 40^{o}C, tropft dann bei Raumtemperatur 11,5 g (80 mmol) Methyliodid in 10 ml THF zu und läßt 2 Tage stehen. Man verdünnt mit 300 ml Essigester, wäscht dreimal mit Wasser, trocknet über Magnesiumsulfat und engt im Vakuum ein. Nach Umkristallisation aus Isopropanol werden 1,9 g (57 %) 6-Methyl-[1,2,5]oxadiazolo[3,4-d]pyrimidin-7(6H)-on-1-oxid erhalten.
Fp. 136 - 138^{o}C
¹³C-NMR (DMSO): δ = 33,0 (q), 103,0 (s), 152,0 (s), 155,2 (d), 158,5 (s)

### Beispiel 9:

### 6-(N,N-Dimethylaminocarbonylmethyl)-[1,2,5]oxadiazolo[3,4-d]pyrimidin-7(6H)-on-1-oxid

4,6 g (30 mmol) 7-Hydroxy-[1,2,5]oxadiazolo[3,4-d]pyrimidin-1-oxid und 0,28 g (35 mmol) Lithiumhydrid werden in 65 ml THF 30 Minuten auf 50^{o}C erwärmt. Dann tropft man eine Lösung von 4,6 g (38 mmol) 2-Chlor-N,N-dimethylacetamid in 10 ml THF zu und erhitzt 4 Stunden unter Rückfluß. Das Lösungsmittel wird am Rotationsverdampfer vollständig abgezogen und der Rückstand wird 2 Stunden mit 30 ml Wasser gerührt. Man saugt den Niederschlag ab, kristallisiert aus THF um und erhält 2,4 g (33 %) 6-(N,N-Dimethylaminocarbonylmethyl)[1,2,5]oxadiazolo[3,4-d]pyrimidin-7(6H)-on-1-oxid.
Fp. 229 - 230^{o}C

Analog den vorstehenden Arbeitsvorschriften wurden die folgenden Beispiele synthetisiert. Die Strukturen wurden durch Vollanalyse, sowie ¹H- und ¹³C-NMR-Spektroskopie bestätigt.

### Beispiel 10:

### 7-Hydroxyamino-5-methoxy-[1,2,5]oxadiazolo-[3,4-d]pyrimidin-1-oxid

Fp. 179^{o}C

### Beispiel 11:

### 7-n-Butylamino-5-methoxy-[1,2,5]oxadiazolo-[3,4-d]pyrimidin-1-oxid

Fp. 77 - 79^{o}C

### Beispiel 12:

### 7-(2-Hydroxyethylamino)-[1,2,5]oxadiazolo-[3,4-d]pyrimidin-1-oxid

Fp. 117 - 119^{o}C

### Beispiel 13:

### 5-Methoxy-7-(N-Methylpiperazino)-[1,2,5]oxadiazolo[3,4-d]pyrimidin-1-oxid

Fp. 155 - 156^{o}C

### Beispiel 14:

### 7-(N,N-Di-(2-hydroxyethyl)amino)-5-methoxy-[1,2,5]oxadiazolo[3,4-d]pyrimidin-1-oxid

Fp. 160 - 161^{o}C

### Beispiel 15:

### 6-Ethyl-4-methyl-[1,2,5]oxadiazolo[3,4-d]pyrimidin-5,7(4H,6H)-dion-1-oxid

Fp. 160 - 162^{o}C

### Beispiel 16:

### 4-Benzyl-6-ethyl-[1,2,5]oxadiazolo-[3,4-d]pyrimidin-5,7(4H,6H)-dion-1-oxid

Fp. 137 - 139^{o}C

### Beispiel 17:

### 6-Benzyl-[1,2,5]oxadiazolo[3,4-d]pyrimidin-7(6H)-on-1-oxid

Fp. 165 - 170^{o}C

### Beispiel 18:

### 6-Morpholinocarbonylmethyl-[1,2,5]oxadiazolo[3,4-d]pyrimidin-7(6H)-on-1-oxid

Fp. 80 - 85^{o}C

### Beispiel 19:

### 6-Ethoxycarbonylmethyl-[1,2,5]oxadiazolo-[3,4-d]pyrimidin-7(6H)-on-1-oxid

Fp. 163 - 165^{o}C

### Beispiel 20:

### 5-Methoxy-7-(2-methoxyethylamino)-[1,2,5]oxadiazolo[3,4-d]pyrimidin-1-oxid

Fp. 130 - 131^{o}C

### Beispiel 21:

### 7-(2-Hydroxyethylamino)-5-methoxy-[1,2,5]oxadiazolo[3,4-d]pyrimidin-1-oxid

Fp. 143 - 144^{o}C

### Beispiel 22:

### 7-(2-(3,4-Dimethoxyphenyl)ethylamino)5-methoxy-[1,2,5]oxadiazolo[3,4-d]pyrimidin-1-oxid

Fp. 126 - 128^{o}C

### Beispiel 23:

### 6-(3-Picolyl)-[1,2,5]oxadiazolo[3,4-d]pyrimidin-7-(6H)-on -1-oxid-hydrochlorid

5,0 g (33mmol) 7-Hydroxy-[1,2,5]oxadiazolo[3,4-d]-pyrimidin-1-oxid und 0,3 g (38mmol) Lithiumhydrid werden in 70 ml THF 30 min auf 50^{o}C erwärmt. Nach Zugabe einer Lösung von 5,9 g (46mmol) 3-Picolylchlorid in 30 ml DMF erhitzt man 2h auf 60^{o}C. Die Lösungsmittel werden am Rotationsverdampfer vollständig abgezogen, und der Rückstand wird mehrfach mit THF digeriert. Aus den vereinigten THF-Extrakten fällt man das Produkt durch Zugabe etherischer Salzsäure aus und kristalliert aus Methanol um. Man erhält 1 g 6-(3-Picolyl)-[1,2,5]oxadiazolo[3,4-d]pyrimidin-7(6H)-on-1-oxid-hydrochlorid.
Fp. 205 - 207^{o}C

## Patentansprüche

1. Pyrimidofuroxane der allgemeinen Formel I 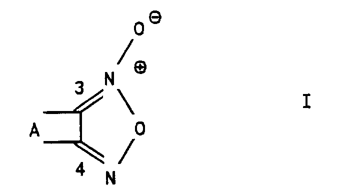 worin 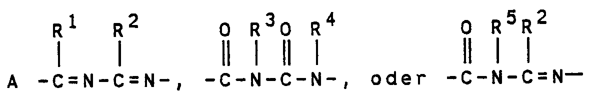 bedeutet, wobei jeweils das Stickstoffatom über das C-4 und das Kohlenstoffatom über das C-3 des Furoxanrings gebunden ist;
R¹ -NHR⁶, -NR⁷R⁸, -N X, -NHOH, -NH(CH₂)ₙY,
-N[(CH₂)ₙY]₂ oder -NH(CH₂)ₘZ bedeutet;
R² Wasserstoff oder -OR⁷ bedeutet;
R³ (C₂-C₄)-Alkyl bedeutet;
R⁴ Wasserstoff, (C₁-C₆)-Alkyl, (C₅-C₇)-Cycloalkyl,
-CH₂Z, -CH₂COOR⁷, -CH₂CONR⁷R⁸, -CH₂CON X oder
-(CH₂)ₙY bedeutet;
R⁵ eine der Bedeutungen von R⁴ mit Ausnahme von Wasserstoff hat;
R⁶ (C₁-C₆)-Alkyl oder (C₅-C₇)-Cycloalkyl bedeutet;
R⁷ und R⁸ unabhängig voneinander (C₁-C₄)-Alkyl bedeutet;
X -(CH₂)ₚ-, -(CH₂)₂-O-(CH₂)₂- oder 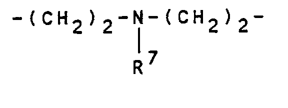 bedeutet;
Y -OH, -OR⁷ oder -NR⁷R⁸ bedeutet;
Z Aryl oder Heteroaryl, die gegebenenfalls auch substituiert sein können, bedeutet;
n für 2, 3 oder 4 steht;
m für 1 oder 2 steht; und
p für 4, 5 oder 6 steht,
sowie deren pharmakologisch annehmbare Säureadditionsverbindungen.

2. Pyrimidofuroxane gemäß Anspruch 1, dadurch gekennzeichnet, daß R¹ 2-(Pyridylmethyl)amino, 3-(Pyridylmethyl)amino, 2-Hydroxyethylamino, 2-Methoxyethylamino, 2-Diethylaminoethylamino, 2-(3,4-Dimethoxyphenyl)ethylamino, n-Butylamino, N,N-Di-(2-hydroxyethyl)amino, N-Methylpiperazino oder Morpholino, bedeutet.

3. Pyrimidofuroxane gemäß Anspruch 1 und/oder 2, dadurch gekennzeichnet, daß R² Wasserstoff, Methoxy oder Ethoxy bedeutet.

4. Pyrimidofuroxane gemäß Anspruch 1, dadurch gekennzeichnet, daß R³ Ethyl bedeutet.

5. Pyrimidofuroxane gemäß Anspruch 1 und/oder 4, dadurch gekennzeichnet, daß R⁴ Wasserstoff, Methyl, Benzyl, Ethoxycarbonylmethyl, Dimethylaminocarbonylmethyl, Morpholinocarbonylmethyl, 2-Pyridylmethyl oder 3-Pyridylmethyl, bedeutet.

6. Pyrimidofuroxane gemäß Anspruch 1 und/oder 3, dadurch gekennzeichnet, daß R⁵ Methyl, Benzyl, Ethoxycarbonylmethyl, Dimethylaminocarbonylmethyl, Morpholinocarbonylmethyl, 2-Pyridylmethyl oder 3-Pyridylmethyl, bedeutet.

7. Verfahren zur Herstellung von Pyrimidofuroxanen der allgemeinen Formel Ia 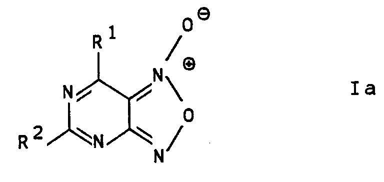 worin R¹ und R² wie in Anspruch 1 angegeben definiert sind, dadurch gekennzeichnet, daß eine Verbindung der allgemeinen Formel II 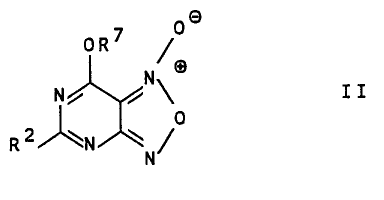 worin R⁷ wie in Anspruch 1 angegeben definiert ist, mit einem Amin der allgemeinen Formel HR¹ umgesetzt wird.

8. Verfahren zur Herstellung von Pyrimidofuroxanen der allgemeinen Formel Ib 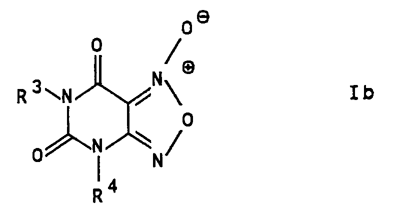 worin R³ und R⁴ wie in Anspruch 1 angegeben definiert sind, dadurch gekennzeichnet, daß gemäß folgendem Schema Verbindungen der allgemeinen Formel VII durch Erhitzen in einem hochsiedenden organischen Lösungsmittel in Verbindungen der allgemeinen Formel Ib/1 überführt werden und gegebenenfalls diese zu Verbindungen der allgemeinen Formel Ib/2, worin R^{4'} mit Ausnahme von Wasserstoff wie R⁴ in Anspruch 1 definiert ist, umgesetzt werden durch basenkatalysierte Alkylierung mit Verbindungen der allgemeinen Formel R^{4'}Hal, worin Hal Chlor, Brom, Jod, Mesylat oder Tosylat bedeutet.

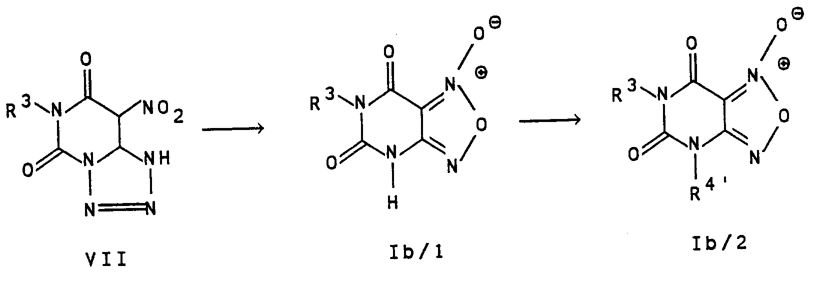

9. Verfahren zur Herstellung von Pyrimidofuroxanen der allgemeinen Formel Ic 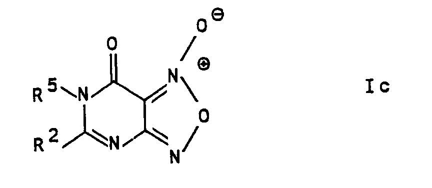 worin R² und R⁵ wie in Anspruch 1 angegeben definiert sind, dadurch gekennzeichnet, daß eine Verbindung der allgemeinen Formel XI 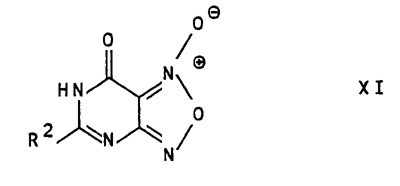 mit einer Verbindung der allgemeinen Formel R⁵Hal, worin R⁵ wie in Anspruch 1 angegeben definiert ist und Hal Chlor, Brom, Jod, Mesylat oder Tosylat bedeutet, unter Basenkatalyse umgesetzt wird.

10. Pharmazeutisches Präparat, dadurch gekennzeichnet, daß es ein Pyrimidofuroxan der allgemeinen Formel I gemäß einem oder mehreren der Ansprüche 1 bis 6, oder ein pharmakologisch annehmbares Säureadditionssalz davon als Wirkstoff zusammen mit pharmazeutisch annehmbaren Träger- und Zusatzstoffen und gegebenenfalls noch ein oder mehrere andere pharmakologische Wirkstoffe enthält.

11. Verwendung eines Pyrimidofuroxans der allgemeinen Formel I gemäß einem oder mehreren der Ansprüche 1 bis 6 oder eines pharmakologisch annehmbaren Säureadditionssalzes davon zur Bekämpfung und Vorbeugung von Erkrankungen des kardiovaskulären Systems, insbesondere zur Bekämpfung und Vorbeugung von Angina pectoris, und zur Behandlung erektiler Dysfunktionen.
